# EUROPÄISCHE PATENTSCHRIFT

(11) **EP 3 227 615 B1**
(45) Veröffentlichungstag und Bekanntmachung des Hinweises auf die Patenterteilung: **22.05.2019**
(21) Anmeldenummer: 15801845.7
(22) Anmeldetag: 30.11.2015
(51) Int. Cl.: F24F 3/16, A61L 9/00, H05H 1/48

(54) **LÜFTUNGSANLAGE UND VERFAHREN ZU DEREN BETRIEB**
VENTILATION SYSTEM AND METHOD FOR OPERATING SAME
SYSTÈME DE VENTILATION ET PROCÉDÉ PERMETTANT DE FAIRE FONCTIONNER LEDIT SYSTÈME

(30) Priorität: 01.12.2014 DE 102014117624
(43) Veröffentlichungstag der Anmeldung: 11.10.2017
(73) Patentinhaber: PlasmaTreat GmbH, 33803 Steinhagen (DE)
(72) Erfinder: BUSKE, Christian, 33619 Bielefeld (DE); HASSE, Daniel, 33104 Paderborn (DE)
(74) Vertreter: Cohausz & Florack
(86) Internationale Anmeldenummer: PCT/EP2015/078033
(87) Internationale Veröffentlichungsnummer: WO 2016/087357

(56) Entgegenhaltungen:
- EP-A1- 1 666 152
- EP-A1- 2 301 588
- EP-A2- 2 154 440
- WO-A1-2007/061295
- DE-A1- 10 313 385
- DE-A1-102006 049 320
- DE-U1-202006 016 535
- JP-A- 2008 049 002

## Beschreibung

Die Erfindung betrifft eine Lüftungsanlage, insbesondere eine Klimaanlage, mit einem Leitungssystem, das dazu eingerichtet ist, einen Luftstrom zu leiten und den Luftstrom während eines Normalbetriebs über einen Auslass des Leitungssystems zur Versorgung eines Raums bereitzustellen. Weiterhin betrifft die Erfindung die Verwendung einer solchen Belüftungsanlage sowie ein Verfahren zu deren Betrieb.

Lüftungs- und Klimaanlagen werden insbesondere in der Gebäudetechnik eingesetzt, um Räume mit Frischluft zu versorgen und/oder diese zu klimatisieren. Bei dem Betrieb derartiger Anlagen stellt sich jedoch das Problem, dass diese mit der Zeit verschmutzen oder verkeimen können, wodurch einerseits die Funktionsfähigkeit dieser Anlagen beeinträchtigt wird und andererseits ein erhebliches Gesundheitsrisiko für sich in den belüfteten bzw. klimatisierten Räumen aufhaltende Personen entstehen kann.

Dies ist besonders problematisch, wenn die Lüftungs- bzw. Klimaanlagen zur Belüftung oder Klimatisierung von Operationssälen oder anderen steril zu haltenden Räumen eingesetzt werden. So kann es bereits eine geringe Verkeimung der Belüftungsanlage zu einer Kontamination eines solchen Operationssaals und damit zu einer erhöhten Infektionsgefahr für die dort behandelten Patienten führen, die im Extremfall sogar lebensbedrohlich werden kann.

Aus diesem Grund gibt es in vielen Ländern strenge Hygieneanforderungen an raumlufttechnische Anlagen und Geräte, die in Krankenhäusern eingesetzt werden. In Deutschland werden diese Anforderungen beispielsweise in der DIN 1946 Teil 4 sowie in der VDI-Norm 6022 definiert.

Um eine Verkeimung von Klimaanlagen und Lüftungsanlagen zu verhindern, werden im Stand der Technik regelmäßig Proben an den Anlagen genommen und auf erhöhte Keimwerte untersucht. Wird bei einer solchen Messung eine Verkeimung festgestellt, so wird eine Wartung und Reinigung der entsprechenden Lüftungs- bzw. Klimaanlage in Auftrag gegeben. Dieses Vorgehen hat jedoch mehrere Nachteile. Erstens kann in den Zeiten zwischen den Probennahmen eine beginnende Verkeimung unentdeckt bleiben. Zweitens kann eine Wartung und Reinigung der Klimaanlage häufig nicht sofort erfolgen, wodurch die Reinigung verzögert und das Infektionsrisiko erhöht wird. Schließlich erfordert die Reinigung und Wartung von Klima- und Lüftungsanlagen, die häufig durch Spezialbetriebe durchgeführt wird, einen nicht unerheblichen Aufwand und erfordert regelmäßig eine zeitweise Stilllegung des durch die Lüftungs- bzw. Klimaanlage versorgten Raumes. Hierdurch kann es beispielsweise zu einer Stilllegung eines Operationssaals für 24 Stunden kommen, wodurch sich hohe Ausfallzeiten und -kosten sowie ggf. Kapazitätsengpässe ergeben. EP2301588 offenbart den Oberbegriff des Anspruchs 1.

DE10313385 offenbart den Oberbegriff des Anspruchs 15.

Vor diesem Hintergrund liegt der vorliegenden Erfindung die Aufgabe zugrunde, eine Lüftungsanlage zur Verfügung zu stellen, mit der die zuvor genannten Probleme zumindest teilweise überwunden werden können.

Diese Aufgabe wird bei einer Lüftungsanlage, insbesondere einer Klimaanlage, mit einem Leitungssystem, das dazu eingerichtet ist, einen Luftstrom zu leiten und den Luftstrom während eines Normalbetriebs über einen Auslass des Leitungssystems zur Versorgung eines Raums bereitzustellen, erfindungsgemäß zumindest teilweise dadurch gelöst, dass die Lüftungsanlage eine Erzeugungseinheit aufweist, die dazu eingerichtet ist, während eines Reinigungsbetriebs reaktive Sauerstoffspezies zu erzeugen und diese dem im Leitungssystem geleiteten Luftstrom zuzuführen. Weiterhin wird die zuvor genannte Aufgabe gelöst durch ein Verfahren zum Betrieb der zuvor genannten Lüftungsanlage, insbesondere Klimaanlage, bei dem ein Luftstrom in dem Leitungssystem geleitet und während eines Normalbetriebs über den Auslass zur Versorgung eines Raums bereitgestellt wird, insbesondere einem Raum zugeführt wird, und bei dem während eines Reinigungsbetriebs reaktive Sauerstoffspezies mit der Erzeugungseinheit erzeugt und einem im Leitungssystem geleiteten Luftstrom zugeführt werden.

Es wurde festgestellt, dass sich die Hygiene und der Betrieb von Lüftungs- bzw. Klimaanlagen verbessern lassen, indem eine Erzeugungseinheit zur Erzeugung reaktiver Sauerstoffspezies unmittelbar in eine solche Lüftungs- bzw. Klimaanlage integriert wird. Auf diese Weise kann die Lüftungs- bzw. Klimaanlage, in regelmäßigen Abständen desinfiziert oder sogar sterilisiert werden, so dass eine Verkeimung beseitigt wird oder gar nicht erst entstehen kann.

Unter einer Desinfektion wird gemäß DIN EN 1040 eine Reduktion der Keimzahl um mindestens 5 dezimal logarithmische Stufen für Bakterien und um mindestens 4 dezimal logarithmische Stufen für Viren verstanden. Unter einer Sterilisation wird gemäß DIN EN 556 eine Reduktion der Keimzahl um mindestens 6 dezimal logarithmische Stufen (für Viren und Bakterien) verstanden.

Die Lüftungsanlage weist ein Leitungssystem auf, das dazu eingerichtet ist, einen Luftstrom zu leiten. Typischerweise umfasst ein solches Leitungssystem Rohrleitungen, mit denen ein Luftstrom von einem Ort, beispielsweise einem Wärmetauscher einer Klimaanlage, einer zentralen Luftversorgung oder einer Luftansaugöffnung, beispielsweise an einer Gebäudeaußenseite, zu einem anderen Ort geleitet werden kann, insbesondere einer Auslassöffnung, die in einen zu versorgenden Raum mündet. Das Leitungssystem kann weiterhin Luftantriebsmittel wie Rotoren oder Düsen aufweisen, um den Luftstrom im Leitungssystem zu erzeugen bzw. aufrechtzuerhalten.

Das Leitungssystem ist weiterhin dazu eingerichtet, den Luftstrom während eines Normalbetriebs über einen Auslass des Leitungssystems zur Versorgung eines Raumes bereitzustellen. Hierzu weist das Leitungssystem typischerweise eine Auslassöffnung auf, mit der das Leitungssystem an eine entsprechende Durchbrechung in einem zu versorgenden Raum angeschlossen werden kann, beispielsweise an eine Lüftungsöffnung in einer Wand des Raums. Unter dem Normalbetrieb wird vorliegend der normale Lüftungsbetrieb bzw. Klimatisierungsbetrieb der Lüftungs- bzw. Klimaanlage verstanden.

Die Lüftungsanlage weist eine Erzeugungseinheit auf, die dazu eingerichtet ist, während eines Reinigungsbetriebs reaktive Sauerstoffspezies zu erzeugen. Unter einem Reinigungsbetrieb wird vorliegend ein zu dem Normalbetrieb alternativer Betriebsmodus der Lüftungsanlage verstanden, der zur Reinigung, Desinfektion bzw. Sterilisation der Lüftungsanlage dient.

Unter reaktiven Sauerstoffspezies (Reactive Oxygen Species - ROS) werden reaktive Formen des Sauerstoffs verstanden, die eine auf Mikroorganismen schädliche Wirkung haben und daher zur Desinfektion und Sterilisation geeignet sind. Typische Beispiele für reaktive Sauerstoffspezies sind: Hyperoxid-Anionen (O₂⁻), Superoxid-Anion Radikale (O₂·⁻), Hydroxy-Radikale (HO·), Hydroperoxid-Radikale (HOO·), Peroxyradikale (ROO·), Alkylradikale (RO·) Wasserstoffperoxid (H₂O₂), Hydroperoxid (ROOH), Ozon (O₃), Hypochlorid-Anionen (OCl⁻), Singulett-Sauerstoff (¹O₂) oder auch Stickstoffverbindungen, wie NO₂⁺, NO⁻ etc. Vorzugsweise werden während des Reinigungsbetriebs vor allem Peroxide und Ozon als reaktive Sauerstoffspezies erzeugt, die eine gute Desinfektions- und Sterilisationswirkung haben.

Der vorliegend verwendete Begriff "Mikroorganismen" umfasst außer Mikroorganismen im eigentlichen Sinn auch zelluläres oder nichtzelluläres biologisches Material, das fähig ist, sich zu vermehren oder genetisches Material weiterzugeben. Weiterhin ist entsprechend CEN 12740 auch biologisches Material eingeschlossen, das Infektionen, Allergien auslösen kann oder toxisch wirksam ist. Gemäß diesen Definitionen zählen Viren, Viroide, Parasiten, Zellen aus mehrzelligen pflanzlichen und tierischen Organismen, Prionen, Plasmide auch unter den Begriff Mikroorganismen.

Durch die Integration der Erzeugungseinheiten in die Lüftungsanlage können diese reaktiven Sauerstoffspezies bei Bedarf direkt in gewünschter Menge in der Lüftungsanlage erzeugt werden. Dies ist insbesondere vorteilhaft gegenüber einer Bevorratung und bedarfsmäßiger Freigabe reaktiver Sauerstoffspezies, da die für die Desinfektion bzw. Sterilisation besonders wirksamen Sauerstoffspezies eine kurze Halbwertszeit aufweisen und sich bei Lagerung schnell in weniger reaktive Spezies umwandeln. Insbesondere kann die Lüftungsanlage so eingerichtet werden, dass die Verbreitung der ROS möglichst optimal ausfällt, indem sowohl die Anzahl als auch die Position der Erzeugungseinheiten angepasst werden kann. Dadurch wird eine lokale und optimale Verbreitung der ROS gewährleistet.

Im Folgenden werden verschiedene Ausführungsformen der Lüftungsanlage und des Verfahrens zu deren Betrieb beschrieben, wobei die einzelnen Ausführungsformen sowohl für die Lüftungsanlage als auch für das Verfahren anwendbar sind. Zudem sind die einzelnen Ausführungsformen untereinander kombinierbar.

Bei einer ersten Ausführungsform weist die Lüftungsanlage eine Steuerung auf, die dazu eingerichtet ist, die Erzeugungseinheit derart zu steuern, dass während eines Reinigungsbetriebs in einem im Leitungssystem geleiteten Luftstrom eine Konzentration an reaktiven Sauerstoffspezies von mindestens 7 g/m³, vorzugsweise von mindestens 10 g/m³ erreicht wird. Vorzugsweise ist die Steuerung dazu eingerichtet, die Erzeugungseinheit derart zu steuern, dass während eines Reinigungsbetriebs in einem im Leitungssystem geleiteten Luftstrom eine Ozon-Konzentration von mindestens 7 g/m³, vorzugsweise von mindestens 10 g/m³ erreicht wird. Es wurde festgestellt, dass bei einer Konzentration der reaktiven Sauerstoffspezies bzw. insbesondere bei einer Konzentration des Ozons von mindestens 7 g/m³ eine ausreichende Desinfektion der Lüftungsanlage erreicht werden kann. Mit einer Konzentration der reaktiven Sauerstoffspezies bzw. des Ozons von mindestens 10 g/m³ kann eine ausreichende Sterilisation der Lüftungsanlage erreicht werden.

Bei einer weiteren Ausführungsform weist die Lüftungsanlage eine Steuerung auf, die dazu eingerichtet ist, die Lüftungsanlage zu vorgegeben Zeiten und/oder für vorgegebene Dauern im Reinigungsbetrieb zu betreiben. Beispielsweise kann die Steuerung dazu eingerichtet sein, die Lüftungsanlage für einen Zeitraum von beispielsweise einer Stunde während der Nacht zu betreiben, so dass die Lüftungsanlage jede Nacht gereinigt und sterilisiert wird und damit am nächsten Morgen betriebsbereit und sauber zur Verfügung steht. Auf diese Weise wird ein wartungsarmer und reibungsloser Betrieb der Lüftungsanlage ermöglich. Zudem können Ausfallzeiten von durch die Lüftungsanlage versorgten Räumen vermieden werden.

Bei einer weiteren Ausführungsform ist die Steuerung dazu eingerichtet, die Erzeugungseinrichtung während des Reinigungsbetriebs phasenweise zu betreiben. Unter einem phasenweisen Betrieb wird verstanden, dass die Erzeugungseinheit nicht während des gesamten Reinigungsbetriebs reaktive Sauerstoffspezies erzeugt, sondern dass der Reinigungsbetrieb auch mindestens einen, vorzugsweise mehrere Abklingzeiträume umfasst, während denen die Erzeugungseinheit keine reaktiven Sauerstoffspezies erzeugt. Es wurde festgestellt, dass die reaktiven Sauerstoffspezies eine gewisse Lebensdauer aufweisen, bevor sie soweit umgesetzt oder umgewandelt sind, dass ihre Konzentration für eine weitere effektive Desinfektion nicht mehr ausreicht. Daher ist es nicht erforderlich, die Erzeugungseinrichtung während des gesamten Reinigungsbetriebs zu betreiben, so dass Energiekosten eingespart werden können.

Vorzugsweise kann der Reinigungsbetrieb eine Mehrzahl von Aktivierungszeiträumen aufweisen, während denen mit der Erzeugungseinheit reaktive Sauerstoffspezies erzeugt werden, wobei die Aktivierungszeiträume von Abklingzeiträumen unterbrochen werden, während denen keine weiteren Sauerstoffspezies erzeugt werden. Eine durchgehend für die Desinfektion ausreichende Konzentration an reaktiven Sauerstoffspezies wurde in Versuchen beispielsweise bei Aktivierungszeiträumen von jeweils 10 Minuten und daran anschließenden Abklingzeiträumen von jeweils 20 Minuten erreicht.

Bei einer weiteren Ausführungsform ist das Leitungssystem zwischen einem Zuluftmodus und einem Umluftmodus umschaltbar, wobei das Leitungssystem im Zuluftmodus dazu eingerichtet ist, einen im Leitungssystem geleiteten Luftstrom über den Auslass zur Versorgung eines Raums bereitzustellen, und wobei das Leitungssystem im Umluftmodus dazu eingerichtet ist, einen im Leitungssystem geleiteten Luftstrom innerhalb des Leitungssystems zu zirkulieren. Bei einer entsprechenden Ausführungsform des Verfahrens wird während des Reinigungsbetriebs der Luftstrom innerhalb des Leitungssystems zirkuliert. Vorzugsweise weist die Lüftungsanlage eine Steuerung auf, die dazu eingerichtet ist, das Leitungssystem während des Reinigungsbetriebs in den Umluftmodus zu schalten.

Es wurde festgestellt, dass eine effektivere Reinigung der Lüftungsanlage und der Klimaanlage insbesondere dadurch erreicht werden kann, dass der mit reaktiven Sauerstoffspezies angereicherte Luftstrom innerhalb des Leitungssystems zirkuliert wird, ohne dass der Luftstrom aus dem Leitungssystem heraus tritt. Einerseits lässt sich hierdurch die Dauer des Reinigungsbetriebs bzw. die Dauer von Aktivierungszeiträumen verkürzen. Andererseits wird verhindert, dass reaktive Sauerstoffspezies, insbesondere Ozon, aus dem Leitungssystem heraus gelangen und so zu einer gesundheitlichen Belastung oder einer Geruchsbelastung im zu versorgenden Raum führen.

Zum Umschalten zwischen dem Zuluft- und dem Umluftmodus kann das Leitungssystem beispielsweise ansteuerbare Luftleitelemente wie verschließbare Öffnungen oder Klappen aufweisen, mit denen der im Leitungssystem geleitete Luftstrom durch verschiedene Leitungsabschnitte geleitete werden kann. Durch Öffnen und Schließen bestimmter Luftleitelemente kann das Leitungssystem so zwischen einem Zuluft- und einem Umluftmodus hin- und hergeschaltet werden.

Der Reinigungsbetrieb kann alternativ auch im Zuluftmodus betrieben werden. Weist die Lüftungsanlage beispielsweise neben einem Auslass auch einen Einlass auf, der dazu eingerichtet ist, einen Luftstrom aus dem zu versorgenden Raum abzusaugen, so kann der Kreislauf des mit reaktiven Sauerstoffspezies angereicherte Luftstroms auch über den zu versorgenden Raum geschlossen werden, so dass der Luftstrom vom Leitungssystem durch den Auslass in den zu versorgenden Raum und von dort durch den Einlass wieder in das Leitungssystem gelangt. Auf diese Weise kann insbesondere auch eine zumindest teilweise Desinfektion oder sogar Sterilisation des zu versorgenden Raums erreicht werden, beispielsweise eines Raumes eines Gebäudes, insbesondere eines Operationssaals, eines Quarantäne-Raumes auf einer Isolierstation (Isolationszimmer), eines Reinraums oder eines Laborraumes, oder eines Innenraumes eines geschlossenen Geräts, wie zum Beispiel eines Innenraumes eines Laborgeräts (insb. eines Brutschranks für Zell- und Gewebekulturen) oder eines Haushaltsgeräts (insb. eines Kühlschranks etc.).

Bei einer weiteren Ausführungsform ist an der Erzeugungseinheit eine Einspeisung vorgesehen, die dazu eingerichtet ist, einen Zusatzstoff in die Erzeugungseinheit einzuspeisen. Insbesondere kann die Einspeisung dazu eingerichtet sein, Wasser oder Wasserdampf oder einen anderen Zusatzstoffs bzw. ein Additiv, beispielsweise als Pulver oder Aerosol, in die Erzeugungseinheit einzuspeisen. Über die Einspeisung können die Arten, die Mengen und/oder die Verteilung der beim Betrieb der Erzeugungseinheit erzeugten reaktiven Sauerstoffspezies beeinflusst werden.

Bei einer weiteren Ausführungsform ist die Erzeugungseinheit dazu eingerichtet, reaktive Sauerstoffspezies mittels einer elektrischen Entladung in einem Arbeitsgas zu erzeugen, vorzugsweise mittels einer dielektrisch behinderten Entladung. Durch eine solche elektrische Entladung lassen sich reaktive Sauerstoffspezies in ausreichender Konzentration bedarfsgemäß erzeugen, ohne dass es neben einer elektrischen Versorgung aufwändiger Infrastruktur zum Betrieb der Erzeugungseinheit bedarf. Als besonders geeignet zur Erzeugung reaktiver Sauerstoffspezies hat sich eine dielektrisch behinderte Entladung herausgestellt. Hierbei wird zwischen zwei Elektroden eine hochfrequente Hochspannung angelegt, wobei ein zwischen den Elektroden angeordnetes Dielektrikum eine direkte Entladung zwischen den Elektroden behindert.

Bei einer weiteren Ausführungsform umfasst die Lüftungsanlage eine Vorrichtung zur Reduzierung einer Ozon-Konzentration, die dazu eingerichtet ist, die Ozon-Konzentration des im Leitungssystem geleiteten Luftstroms zu reduzieren, vorzugsweise auf einen Wert unter 0,1 ppm. Als Vorrichtung zur Reduzierung einer Ozon-Konzentration können beispielsweise ein oder mehrere Keramikfilter und/oder Aktivkohlefilter vorgesehen sein.

Weiterhin kommt als Vorrichtung zur Reduzierung einer Ozon-Konzentration auch eine Plasmadüse zur Erzeugung eines atmosphärischen Plasmastrahls in Frage. Daher umfasst die Lüftungsanlage bei einer Ausführungsform eine Plasmadüse zur Erzeugung eines atmosphärischen Plasmastrahls, wobei die Plasmadüse dazu eingerichtet ist, den Plasmastrahl mittels Erzeugung einer Bogenentladung durch Anlegen einer hochfrequenten Hochspannung zwischen zwei Elektroden in einem Arbeitsgas zu erzeugen, und das Leitungssystem und die Plasmadüse sind derart eingerichtet, dass der in dem Leitungssystem geleitete Luftstrom der Plasmadüse als Arbeitsgas zuführbar ist.

Unter einer hochfrequenten Hochspannung wird typischerweise eine Spannung von 1 bis 100 kV, insbesondere von 1 bis 50 kV, bevorzugt von 10 bis 50 kV, bei einer Frequenz von 1 bis 350 kHz, bevorzugt 1 bis 100 kHz, besonders bevorzugt 10 bis 100 kHz, insbesondere von 10 bis 50 kHz verstanden. Die hochfrequente Hochspannung kann eine hochfrequente Wechselspannung, aber auch eine gepulste Gleichspannung sein.

Ein auf diese Weise erzeugter atmosphärischer Plasmastrahl weist eine hohe Reaktivität bei verhältnismäßig geringer Temperatur auf. Weiterhin weist der auf diese Weise erzeugte Plasmastrahl nur eine sehr geringe Ozonkonzentration auf und es wurde erkannt, dass es durch die Erzeugung eines solchen Plasmastrahls möglich ist, die Ozonkonzentration in einem Arbeitsgas zu reduzieren.

Damit kann mit den zuvor beschriebenen Ausführungsformen erreicht werden, dass die Ozonkonzentration in dem im Leitungssystem geleiteten Luftstrom mittels der Vorrichtung zur Reduzierung einer Ozon-Konzentration, insbesondere mittels des Keramik- und/oder Aktivkohlefilters und/oder der Plasmadüse gezielt reduziert wird. Dadurch kann die Ozonkonzentration innerhalb der Lüftungsanlage am Ende eines Reinigungsbetriebs auf einen Wert unterhalb eines bestimmten Grenzwerts, insbesondere auf einen Wert unterhalb von 0,1 ppm Ozon, reduziert werden, so dass bei einem nachfolgenden Normalbetrieb Geruchsbelästigungen und Gesundheitsgefährdungen durch in den zu versorgenden Raum abgegebenes Ozon reduziert werden.

Eine effektive Reduktion der Ozon-Konzentration lässt sich insbesondere durch eine Kombination der Plasmadüse mit mindestens einem Keramik- und/oder Aktivkohlefilter erreichen. Daher umfasst die Lüftungsanlage vorzugsweise sowohl eine Plasmadüse als auch einen oder mehrere Keramik- und/oder Aktivkohlefilter.

Bei einer weiteren Ausführungsform weist die Lüftungsanlage eine Steuerung auf, die dazu eingerichtet ist, die Vorrichtung zur Reduzierung einer Ozon-Konzentration, insbesondere die Plasmadüse für einen vorgegebenen Zeitraum am Ende eines Reinigungsbetriebs zu betreiben. Bei einer entsprechenden Ausführungsform des Verfahrens werden während eines ersten Zeitabschnitts des Reinigungsbetriebs reaktive Sauerstoffspezies mit der Erzeugungseinheit erzeugt und einem im Leitungssystem geleiteten Luftstrom zugeführt und während eines zweiten Zeitabschnitts des Reinigungsbetriebs wird der ins Leitungssystem geleitete Luftstrom zumindest teilweise der Vorrichtung zur Reduzierung einer Ozon-Konzentration, insbesondere einer Plasmadüse zur Erzeugung eines atmosphärischen Plasmastrahls als Arbeitsgas zugeführt.

Auf diese Weise wird gewährleistet, dass die Ozonkonzentration innerhalb der Lüftungsanlage zum Ende des Reinigungsbetriebs reduziert wird. Vorzugsweise wird die Erzeugungseinheit für den vorgegebenen Zeitraum am Ende des Reinigungsbetriebs ausgeschaltet, so dass durch die Erzeugungseinheit kein neues Ozon produziert wird. Der vorgegebene Zeitraum umfasst vorzugsweise eine Zeitspanne im Bereich von 3 bis 20 min., vorzugsweise von 5 bis 10 min.

Bei einer weiteren Ausführungsform ist die Lüftungsanlage als Klimaanlage ausgebildet und weist einen Wärmetauscher auf, der dazu eingerichtet ist, während des Normalbetriebs einen in dem Leitungssystem geleiteten Luftstrom zu kühlen, und die Erzeugungseinheit ist derart angeordnet und eingerichtet, dass mit der Erzeugungseinheit erzeugte Sauerstoffspezies zum Wärmetauscher gelangen.

Bei Klimaanlagen ist das Verkeimungsrisiko insbesondere im Bereich des Wärmetauschers hoch, da dieser zur Verbesserung der Wärmeübertragung eine hohe Oberfläche aufweist, die für Verkeimungen besonders anfällig ist. Insbesondere weisen solche Wärmetauscher typischerweise Kühlkörper wie zum Beispiel Lamellen auf, die eine hohe Oberfläche aufweisen.

Daher ist die Erzeugungseinheit vorzugsweise derart angeordnet und eingerichtet, dass mit der Erzeugungseinheit erzeugte reaktive Sauerstoffspezies, insbesondere in den Bereich der Lamellen bzw. des Kühlkörpers des Wärmetauschers gelangen. Weiter bevorzugt ist die Erzeugungseinheit derart angeordnet und eingerichtet, dass im Reinigungsbetrieb im Bereich des Wärmetauschers, insbesondere im Bereich des Kühlkörpers bzw. der Lamellen, eine Konzentration an reaktiven Spezies, insbesondere an Ozon, von mindestens 7 g/m³, vorzugsweise von mindestens 10 g/m³ erreicht wird.

Bei einer weiteren Ausführungsform weist die Lüftungsanlage eine Mehrzahl von Erzeugungseinheiten auf, die jeweils dazu eingerichtet sind, während eines Reinigungsbetriebs reaktive Sauerstoffspezies zu erzeugen und die erzeugten reaktiven Sauerstoffspezies einem im Leitungssystem geleiteten Luftstrom zuzuführen, und die Lüftungsanlage weist eine Steuerung auf, die dazu eingerichtet ist, die Erzeugungseinheiten während eines Reinigungsbetriebs abwechselnd zu betreiben.

Durch das Vorsehen einer Mehrzahl von Erzeugungseinheiten und der damit bewirkten dezentralen Erzeugung reaktiver Sauerstoffspezies kann erreicht werden, dass die Konzentration an reaktiven Sauerstoffspezies, insbesondere an Ozon, während des Reinigungsbetriebs im gesamten Leitungssystem ausreichend hoch ist, vorzugsweise mindestens 7 g/m³, weiter bevorzugt mindestens 10 g/m³. Insbesondere ist diese Ausführungsform für mehrteilige Leitungssysteme geeignet, in denen mehrere Luftströme bzw. mehrere Wege für die Luftströme bestehen.

Durch die abwechselnde Ansteuerung der Erzeugungseinheiten kann die für den Betrieb der Lüftungsanlage erforderliche Leistung reduziert werden. Indem jeweils nur eine oder eine geringe Zahl von Erzeugungseinheiten gleichzeitig angesteuert wird, beträgt die für den Betrieb der Erzeugungseinheiten erforderliche Leistung weniger als das der Anzahl der Erzeugungseinheiten entsprechende Vielfache der Eingangsleistung einer einzelnen Erzeugungseinheit. Auf diese Weise kann das zur Versorgung der Erzeugungseinheiten vorgesehene Netzteil kleiner dimensioniert werden, so dass die Kosten der Lüftungsanlage reduziert werden.

Bei einer weiteren Ausführungsform weist die Lüftungsanlage eine Steuerung auf, die dazu eingerichtet ist, das oben beschriebene Verfahren zum Betrieb einer Lüftungsanlage oder eine beschriebenen Ausführungsform dieses Verfahrens durchzuführen. Unter einer Steuerung wird vorliegend und in der gesamten Anmeldung insbesondere eine elektronische Steuerung verstanden, die beispielsweise einen Mikroprozessor und einen damit verbundener Speicher mit Befehlen umfasst, wobei die Ausführung der Befehle durch den Prozessor die Durchführung der vorgesehenen Steuerung, insbesondere des oben beschriebenen Verfahrens oder einer Ausführungsform davon veranlasst. Mit einer solchen Steuerung ist der Betrieb der Lüftungsanlage weitgehend automatisierbar, so dass es menschliche Eingriffe oder Wartungen nicht mehr bedarf. Insbesondere kann auf diese Weise ein automatischer Reinigungsbetrieb durchgeführt werden, so dass das Verkeimungsrisiko der Lüftungs- bzw. Klimaanlage reduziert wird.

Die Lüftungsanlage kann stationär fest installiert sein, beispielsweise in einem Gebäude wie einem Krankenhaus oder einem Labor. Alternativ kann die Lüftungsanlage auch mobil ausgeführt sein, um sie beispielsweise an verschiedenen Orten aufstellen und betreiben zu können bzw. um sie an verschiedene Räume anschließen zu können.

Bei einer weiteren Ausführungsform ist der Auslass des Leitungssystems an einer Lüftungsöffnung eines Raums angeschlossen. Bei dieser Ausführungsform handelt es sich also um eine (stationäre oder mobile) Lüftungsanlage in einem installierten Zustand, die an den zu versorgenden Raum angeschlossen ist. Bei dem Raum handelt es sich vorzugsweise um einen Raum eines Gebäudes, insbesondere um einen Operationssaal, einen Quarantäne-Raum auf einer Isolierstation (Isolationszimmer), einen Reinraum oder um einen Laborraum. Weiterhin kann es sich bei dem Raum auch um einen Innenraum eines geschlossenen Geräts handeln, wie zum Beispiel um einen Innenraum eines Laborgeräts (insb. eines Brutschranks für Zell- und Gewebekulturen) oder eines Haushaltsgeräts (insb. eines Kühlschranks etc.). Bei derartigen Räumen ist eine Verunreinigung oder Verkeimung von Lüftungsanlagen bzw. Klimaanlagen besonders kritisch, so dass die vorliegende Erfindung besonderes für solche Räume Vorteile bietet. Weiterhin verursacht ein Ausfall derartiger Räume typischerweise erhebliche Kosten, so dass durch eine Einsparung von Wartungszeiten durch den vorzugsweise automatisch ablaufenden Reinigungsbetrieb der Lüftungsanlage erhebliche Kosten eingespart werden können. Insbesondere kann die Lüftungsanlage auch zur zumindest teilweisen Desinfektion oder Sterilisation des angeschlossenen Raums verwendet werden.

Entsprechend wird die oben genannte Aufgabe erfindungsgemäß weiterhin gelöst durch die Verwendung der zuvor beschriebenen Lüftungsanlage bzw. einer der beschriebenen Ausführungsform davon zur Belüftung und/oder Klimatisierung eines Raumes eines Gebäudes, insbesondere eines Operationssaals, eines Quarantäne-Raumes auf einer Isolierstation (Isolationszimmer), eines Reinraums oder eines Laborraumes, oder eines Innenraumes eines geschlossenen Geräts, wie zum Beispiel eines Innenraumes eines Laborgeräts (insb. eines Brutschranks für Zell- und Gewebekulturen) oder eines Haushaltsgeräts (insb. eines Kühlschranks etc.).

Weiterhin wird die oben genannte Aufgabe gelöst durch die Verwendung einer Erzeugungseinheit zur Erzeugung reaktiver Sauerstoffspezies für eine zuvor beschriebene Lüftungsanlage, wobei die Erzeugungseinheit einen Gaseinlass, der zur Einleitung eines Arbeitsgases eingerichtet ist, und einen Gasauslass, der zum Auslass des Arbeitsgases bzw. der reaktiven Sauerstoffspezies eingerichtet ist, aufweist, wobei die Erzeugungseinheit dazu eingerichtet ist, während eines Erzeugungsbetriebs reaktive Sauerstoffspezies mittels einer elektrischen Entladung in einem durch den Gaseinlass eingeleiteten Arbeitsgas zu erzeugen, vorzugsweise mittels einer dielektrisch behinderten Entladung, und wobei die Erzeugungseinheit weiterhin dazu eingerichtet ist, während eines Neutralisierungsbetriebs einen atmosphärischen Plasmastrahl mittels Erzeugung einer Bogenentladung durch Anlegen einer hochfrequenten Hochspannung zwischen zwei Elektroden in dem durch den Gaseinlass eingeleiteten Arbeitsgas zu erzeugen.

Eine solche Erzeugungseinheit ist besonders geeignet, um in die zuvor beschriebene Belüftungsanlage integriert zu werden. Durch die Einrichtung der Erzeugungseinheit zur Erzeugung reaktiver Sauerstoffspezies können mit der Erzeugungseinheit die für die Reinigung bzw. Desinfektion der Lüftungsanlage erforderlichen reaktiven Sauerstoffspezies erzeugt werden. Andererseits kann durch die in dieselbe Erzeugungseinheit integrierte Erzeugungseinheit für einen atmosphärischen Plasmastrahls erreicht werden, dass die Ozonkonzentration innerhalb des Arbeitsgases reduziert wird, so dass beispielsweise am Ende eines Reinigungsbetriebs eine Ozonkonzentration unterhalb des vorgegebenen Grenzwertes erreicht werden kann.

Bei einer Ausführungsform der Erzeugungseinheit weist diese einen ersten Erzeugungsabschnitt auf, der dazu eingerichtet ist, während eines Erzeugungsbetriebs reaktive Sauerstoffspezies mittels einer elektrischen Entladung in einem durch den Gaseinlass eingeleiteten Arbeitsgas zu erzeugen, vorzugsweise mittels einer dielektrisch behinderten Entladung, und die Erzeugungseinheit weist in einem zwischen dem ersten Erzeugungsabschnitt und dem Gasauslass angeordneten zweiten Erzeugungsabschnitt auf, der dazu eingerichtet ist, während eines Neutralisierungsbetriebs einen atmosphärischen Plasmastrahl mittels Erzeugung einer Bogenentladung durch Anlegen einer hochfrequenten Hochspannung zwischen zwei Elektroden in dem durch den Gaseinlass eingeleiteten Arbeitsgas zu erzeugen.

Die oben beschriebene Erzeugungseinheit bzw. eine Ausführungsform davon wird vorzugsweise als Erzeugungseinheit der oben beschriebenen Lüftungs- bzw. Klimaanlage verwendet. Vorzugsweise ist die Steuerung der Lüftungsanlage dazu eingerichtet, in einem ersten Zeitabschnitt eines Reinigungsbetriebs der Lüftungsanlage die Erzeugungseinheit im Erzeugungsbetrieb zu betreiben und in einem zweiten Zeitabschnitt des Reinigungsbetriebs, vorzugsweise am Ende eines Reinigungsbetriebs, die Erzeugungseinheit im Neutralisierungsbetrieb zu betreiben.

Weitere Merkmale und Vorteile der Erfindung ergeben sich aus der folgenden Beschreibung mehrerer Ausführungsbeispiele, wobei auf die beigefügte Zeichnung Bezug genommen wird.

In der Zeichnung zeigen
- Fig. 1a-b: ein erstes Ausführungsbeispiel der Lüftungsanlage und des Verfahrens zu deren Betrieb in schematischer Darstellung,
- Fig. 2a-b: ein zweites Ausführungsbeispiel der Lüftungsanlage und des Verfahrens zu deren Betrieb in schematischer Darstellung,
- Fig. 3: eine Erzeugungseinheit zur Erzeugung reaktiver Sauerstoffspezies in schematischer Querschnittsdarstellung,
- Fig. 4: eine Plasmadüse zur Erzeugung eines atmosphärischen Plasmastrahls in schematischer Querschnittsdarstellung,
- Fig. 5: ein Ausführungsbeispiel einer Erzeugungseinheit zur Erzeugung reaktiver Sauerstoffspezies, insbesondere zur Verwendung mit einer Lüftungsanlage, und
- Fig. 6: ein Steuerungsdiagramm entsprechend eines Ausführungsbeispiels des Verfahrens zum Betrieb einer Lüftungsanlage.

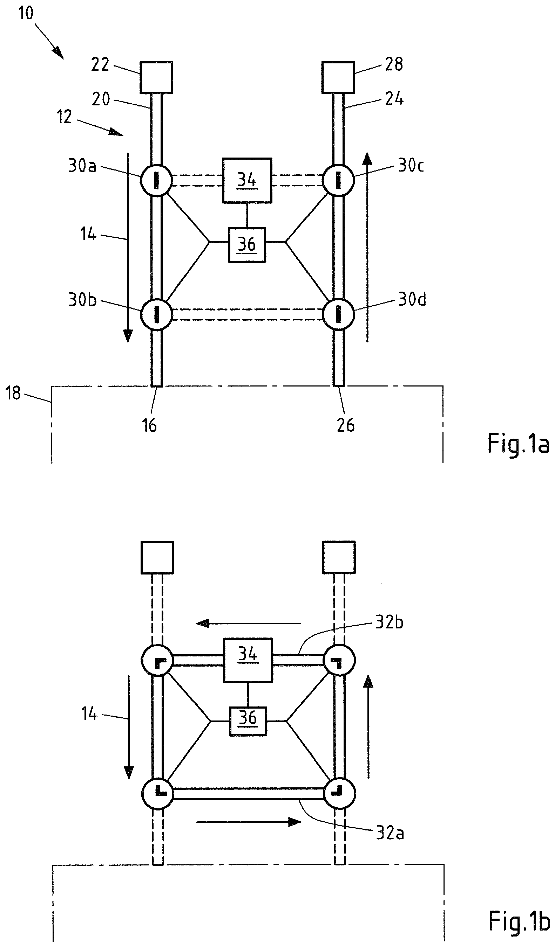

Die Fig. 1a-b zeigen ein erstes Ausführungsbeispiel einer Lüftungsanlage sowie ein Verfahren zu deren Betrieb in schematischer Darstellung.

Die Lüftungsanlage 10 weist ein Leitungssystem 12 auf, das dazu eingerichtet ist, einen Luftstrom 14 zu leiten und den Luftstrom während eines Normalbetriebs über einen Auslass 16 des Leitungssystems 12 zur Versorgung eines Raums 18 bereitzustellen.

Zu diesem Zweck umfasst das Leitungssystem 12 eine erste Leitung 20, die von einer Ansaugöffnung 22 zum Auslass 16 verläuft, der beispielsweise an einer Lüftungsöffnung des Raums 18 angeschlossen sein kann. Daneben umfasst das Leitungssystem 12 eine zweite Leitung 24, die von einem Einlass 26, der beispielsweise an einer weiteren Lüftungsöffnung des Raum 18 angeschlossen sein kann, bis zur Auslassöffnung 28 verläuft.

Im Normalbetrieb wird der Luftstrom 14 durch einen in der Leitung 20 angeordneten Rotor (nicht dargestellt) durch die Ansaugöffnung 22 angesaugt, durch die Leitung 20 geleitet und als Zuluft über den Auslass 16 in den Raum 18 eingebracht. Der Luftstrom 14 wird als Abluft weiterhin durch einen in der Leitung 24 angeordneten Rotor (nicht dargestellt) durch den Einlass 26 gesaugt, durch die Leitung 24 geleitet und durch die Auslassöffnung 28 aus der Lüftungsanlage heraus geführt 10. Mit der Leitung 20 kann der Raum 18 daher mit frischer Luft versorgt werden und mit der Leitung 24 kann verbrauchte Luft aus dem Raum 18 abgesaugt werden.

Das Leitungssystem 12 kann zwischen einem Zuluftmodus (Fig. 1a) und einem Umluftmodus (Fig. 1b) umgeschaltet werden. Zu diesem Zweck weist das Leitungssystem 12 ansteuerbare Luftleitelemente 30a-d und Verbindungsleitungen 32a-b auf, mit denen der Luftstrom 14 umgeleitet werden kann, so dass dieser innerhalb des Leitungssystems 12 zirkulieren kann. Die Luftleitelemente 30a-d können beispielsweise ansteuerbare Klappen aufweisen, mit denen abwechselnd ein Durchgang zur ersten oder zweiten Leitung 20, 24 oder zu einer der Verbindungsleitungen 32a-b geöffnet bzw. geschlossen werden kann, so dass Bereiche der ersten oder zweiten Leitung 20, 24 bzw. der Verbindungsleitungen 32a-b für den Luftstrom 14 freigegeben bzw. gesperrt werden können.

In den Fig. 1a-b sind die von den Luftleitelementen 30a-d für den Luftstrom 14 freigegebenen Bereiche des Leitungssystems 12 mit durchgehenden Linien und die von den Luftleitelementen 30a-d für den Luftstrom 14 gesperrten Bereiche des Leitungssystems 12 mit gestrichelten Linien dargestellt. Vorzugsweise weist das Leitungssystem einen Rotor in einem Bereich der ersten und/oder zweiten Leitung 20, 24 auf, der sowohl im Zuluftmodus als auch im Umfluftmodus freigegeben ist.

Die Lüftungsanlage 10 weist weiterhin eine Erzeugungseinheit 34 auf, die dazu eingerichtet ist, während eines Reinigungsbetriebes reaktive Sauerstoffspezies zu erzeugen und diese dem im Leitungssystem 12 geleiteten Luftstrom 14 zuzuführen. Bei der Lüftungsanlage 10 ist die Erzeugungseinheit 34 in die Verbindungsleitung 32b integriert.

Weiterhin umfasst die Lüftungsanlage eine Steuerung 36, mit der die Luftleitelemente 30a-d und die Erzeugungseinheit 34 gesteuert werden können. Die Steuerung 36 ist derart eingerichtet, dass die Lüftungsanlage 10 einerseits in einem Normalbetrieb und anderseits in einem Reinigungsbetrieb betrieben werden kann.

Im Normalbetrieb werden die Luftleitelemente 30a-d von der Steuerung 36 wie in Fig. 1a dargestellt geschaltet, so dass die Lüftungsanlage 10 zur Be- und Entlüftung des Raums 18 eingerichtet ist. Im Reinigungsbetrieb werden die Luftleitelemente 30a-d von der Steuerung 36 hingegen wie in Fig. 1b dargestellt geschaltet, so dass der Luftstrom 14 innerhalb des Leitungssystems 12 zirkulieren kann. Weiterhin steuert die Steuerung 36 im Reinigungsbetrieb die Erzeugungseinheit 34 an, so dass diese reaktive Sauerstoffspezies erzeugt und dem zirkulierenden Luftstrom 14 zuführt. Die Erzeugungseinheit 34 wird derart gesteuert, dass im Luftstrom 14 eine Konzentration an reaktiven Sauerstoffspezies, insbesondere an Ozon, von mindestens 7 g/m³ (für eine Desinfektion) bzw. von mindestens 10 g/m³ (für eine Sterilisation) erreicht wird. Auf diese Weise kann eine Desinfektion oder sogar Sterilisation der Lüftungsanlage 10 bewirkt werden.

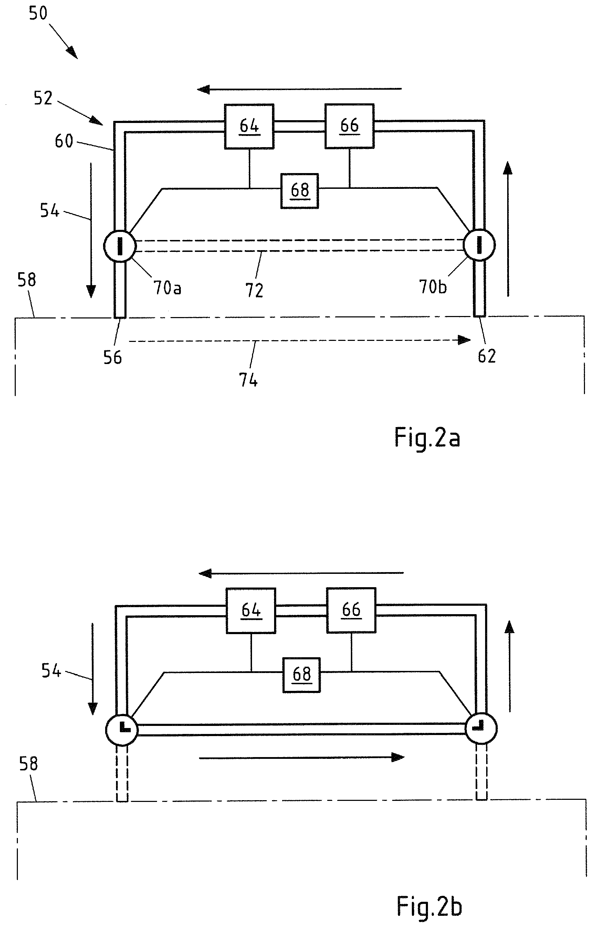

Die Fig. 2a-b zeigen ein weiteres Ausführungsbeispiel einer Lüftungsanlage, die vorliegend als Klimaanlage ausgebildet ist. Die Lüftungsanlage 50 weist ein Leitungssystem 52 auf, das dazu eingerichtet ist, einen Luftstrom 54 während eines Normalbetriebs über einen Auslass 56 des Leitungssystems 52 zur Versorgung eines Raums 58 bereitzustellen.

Die als Klimaanlage ausgebildete Lüftungsanlage 50 ist vorliegend lediglich zur Kühlung ausgebildet. Alternativ kann die Lüftungsanlage zusätzlich auch zur Versorgung mit Frischluft bzw. zur Abführung von Brauchluft ausgebildet.

Das Leitungssystem 52 umfasst eine Hauptleitung 60, die von einem Einlass 62, der beispielsweise an einer Lüftungsöffnung des Raums 58 angeschlossen sein kann, zu einem Wärmetauscher 64 und von dort bis zum Auslass 56 verläuft, der an einer weiteren Lüftungsöffnung des Raums 58 angeschlossen sein kann.

Im Normalbetrieb wird ein Luftstrom 54 mittels eines in der Hauptleitung 60 angeordneten Rotors (nicht dargestellt) durch den Einlass 62 angesaugt, durch die Hauptleitung 60 bis zum Wärmetauscher 64 geleitet, durch den der Luftstrom 54 gekühlt wird, und weiter durch den Auslass 56 in den Raum 58 geleitet, so dass dieser durch den gekühlten Luftstrom 54 klimatisiert wird.

Die Lüftungsanlage 50 weist weiterhin eine Erzeugungseinheit 66 auf, die dazu eingerichtet ist, während eines Reinigungsbetriebs reaktive Sauerstoffspezies zu erzeugen und diese dem Luftstrom 54 zuzuführen. Weiterhin umfasst die Lüftungsanlage 50 noch eine Steuerung 68, die zur Steuerung der Erzeugungseinheit 66 eingerichtet ist. Insbesondere ist die Steuerung 68 dazu eingerichtet, die Erzeugungseinheit 66 während des Reinigungsbetriebs derart zu betreiben, dass im Luftstrom 54 eine Konzentration an reaktiven Sauerstoffspezies, insbesondere an Ozon, von mindestens 7 g/m³ (für eine Desinfektion) bzw. von mindestens 10 g/m³ (für eine Sterilisation) erreicht wird. Auf diese Weise erfolgt im Reinigungsbetrieb eine Desinfektion bzw. sogar Sterilisation der Lüftungsanlage 50. Die Erzeugungseinheit 66 ist vorzugsweise in Strömungsrichtung des Luftstroms 54 vor dem Wärmetauscher 64 angeordnet, so dass die von der Erzeugungseinheit 66 erzeugten reaktiven Sauerstoffspezies in für eine Desinfektion bzw. Sterilisation ausreichenden Konzentration zum Wärmetauscher 64 gelangen und dessen Oberfläche, insbesondere die Oberfläche von Kühlkörpern wie Lamellen des Wärmetauschers 64, desinfizieren bzw. sterilisieren.

Das Leitungssystem 52 ist vorzugsweise zwischen einem Zuluftmodus (Fig. 2a) und einem Umluftmodus (Fig. 2b) umschaltbar. Zu diesem Zweck weist das Leitungssystem 52 ansteuerbare Luftleitelemente 70a-b und eine Verbindungsleitung 72 auf, mit denen der Luftstrom 54 umgeleitet werden kann, so dass dieser innerhalb des Leitungssystems 52 zirkulieren kann. Die Luftleitelemente 70a-b können beispielsweise ansteuerbare Klappen aufweisen, mit denen abwechselnd ein Durchgang zur Hauptleitung 60 oder zur Verbindungsleitung 72 geöffnet bzw. geschlossen werden kann, so dass Bereiche der Hauptleitung 60 bzw. der Verbindungsleitung 72 für den Luftstrom 54 freigegeben bzw. gesperrt werden können. Die Steuerung der Luftleitelemente 70a-b erfolgt über die Steuerung 68.

In den Fig. 2a-b sind die von den Luftleitelementen 70a-d für den Luftstrom 54 freigegebenen Bereiche des Leitungssystems 52 mit durchgehenden Linien und die von den Luftleitelementen 70a-d für den Luftstrom 54 gesperrten Bereiche des Leitungssystems 52 mit gestrichelten Linien dargestellt. Vorzugsweise weist das Leitungssystem einen Rotor in einem Bereich der Hauptleitung 60 auf, der sowohl im Zuluftmodus als auch im Umluftmodus freigegeben ist.

Im Normalbetrieb werden die Luftleitelemente 70a-b von der Steuerung 68 wie in Fig. 2a dargestellt geschaltet, so dass die Lüftungsanlage 50 zur Klimatisierung des Raums 58 eingerichtet ist. Im Reinigungsbetrieb werden die Luftleitelemente 70a-b von der Steuerung 68 hingegen wie in Fig. 2b dargestellt geschaltet, so dass der Luftstrom 54 innerhalb des Leitungssystems 52 zirkulieren kann. Weiterhin steuert die Steuerung 68 im Reinigungsbetrieb die Erzeugungseinheit 66 an, so dass diese reaktive Sauerstoffspezies erzeugt und dem zirkulierenden Luftstrom 54 zuführt. Die Erzeugungseinheit 68 wird derart gesteuert, dass im Luftstrom 54 eine Konzentration an reaktiven Sauerstoffspezies, insbesondere an Ozon, von mindestens 7 g/m³ (für eine Desinfektion) bzw. von mindestens 10 g/m³ (für eine Sterilisation) erreicht wird, so dass eine Desinfektion oder sogar Sterilisation der Lüftungsanlage 50 und insbesondere des Wärmetauschers 64 bewirkt werden kann.

Alternativ kann die Steuerung 68 die Lüftungsanlage 50 auch während des Reinigungsbetriebs im Zuluftmodus (Fig. 2a) betreiben. Der Kreislauf des Luftstroms 54 kann dann über einen im Raum 58 strömenden Luftstrom 74 geschlossen werden. Bei dieser Ausführungsform gelangen die von der Erzeugungseinheit 66 erzeugten reaktiven Sauerstoffspezies auch in den Raum 58, so dass auf diese Weise auch eine zumindest teilwiese Desinfektion oder Sterilisation des Raumes 58 erreicht werden kann. Bei dem Raum 58 kann es sich beispielsweise um einen Raum eines Gebäudes, insbesondere um einen Operationssaal, einen Quarantäne-Raum auf einer Isolierstation (Isolationszimmer), einen Reinraum oder um einen Laborraum handeln. Weiterhin kann es sich bei dem Raum auch um einen Innenraum eines geschlossenen Geräts handeln, wie zum Beispiel um einen Innenraum eines Laborgeräts (insb. eines Brutschranks für Zell- und Gewebekulturen) oder eines Haushaltsgeräts (insb. eines Kühlschranks etc.)einen Operationssaal handeln, der auf diese Weise zumindest teilweise desinfiziert bzw. sterilisiert werden kann.

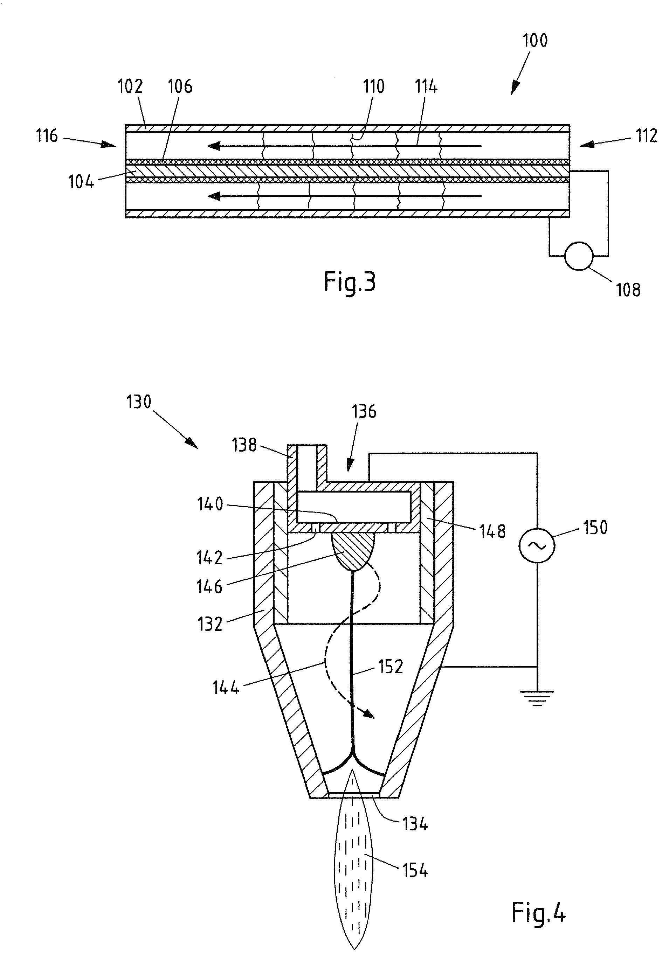

Figur 3 zeigt ein Ausführungsbeispiel einer Erzeugungseinheit 100 zur Erzeugung reaktiver Sauerstoffspezies. Beispielsweise können die Erzeugungseinheiten 34 und 70 der Lüftungsanlagen 10 und 50 wie die Erzeugungseinheit 100 ausgestaltet werden.

Die Erzeugungseinheit 100 weist eine Außenelektrode in Form eines Metallrohrs 102 und eine Innenelektrode in Form eines Metallstabs 104 auf. Die Innenelektrode ist von einer Schicht aus einem dielektrischen Material 106, beispielsweise einer Keramik, umgeben, so dass die Innenelektrode 104 gegenüber der Außenelektrode 102 elektrisch isoliert ist.

Zwischen der Innenelektrode 104 und der Außenelektrode 102 wird mittels einer dafür vorgesehenen Spannungsquelle 108 eine hochfrequente Hochspannung angelegt. Aufgrund der dielektrischen Schicht 106 sind keine direkten elektrischen Entladungen zwischen der Innenelektrode 104 und der Außenelektrode 102 möglich. Es kommt daher im Betrieb der Erzeugungseinheit 100 zu sogenannten dielektrisch behinderten Entladungen 110 zwischen den Elektroden 102, 104.

Die Erzeugungseinheit 100 weist auf der einen Seite einen Gaseinlass 112 auf, durch den ein sauerstoffhaltiges Arbeitsgas 114, insbesondere Luft, in die Erzeugungseinheit 100 gelangen kann. Durch die Wechselwirkung des Arbeitsgases 114 mit den Entladungen 110 entstehen reaktive Sauerstoffspezies, die mit dem übrigen Arbeitsgas 114 aus dem Gasauslass 116 der Erzeugungseinheit 100 austreten.

Die Erzeugungseinheit 100 kann in einer Lüftungsanlage wie der Lüftungsanlage 10 oder 50 beispielsweise derart angeordnet werden, dass der Gaseinlass 112 von einem Luftstrom wie zum Beispiel dem Luftstrom 14 oder 54 angeströmt wird. Zusätzlich kann auch ein Rotor vorgesehen sein, um den Luftstrom in den Gaseinlass 112 zu führen. Die Erzeugungseinheit 100 wird in der Lüftungsanlage weiterhin so angeordnet, dass die aus dem Gasauslass 116 austretenden reaktiven Sauerstoffspezies wieder in das Leitungssystem der Lüftungsanlage bzw. in den dort geleiteten Luftstrom gelangen.

Es wurde festgestellt, dass mit einer Erzeugungseinheit von der in Fig. 3 beschriebenen Art reaktive Sauerstoffspezies in ausreichender Menge und von ausreichender Reaktivität erzeugt werden können, um eine Lüftungsanlage zu desinfizieren oder zu sterilisieren.

Neben anderen reaktiven Sauerstoffspezies entstehen mit der in Fig. 3 dargestellten Erzeugungseinheit 100 auch nicht unerhebliche Mengen an Ozon. Ozon weist in Luft eine relativ lange Halbwertszeit auf, bevor es in andere Produkte umgewandelt wird. Daher kann das Ozon nach Ende eines Reinigungsbetriebs zu einer Gesundheitsbelastung und/oder Geruchsbelästigung im von der Lüftungsanlage versorgten Raum führen.

In einem weiteren Ausführungsbeispiel der Lüftungsanlage, insbesondere der Lüftungsanlagen 10 und 50, kann daher eine Plasmadüse zur Erzeugung eines atmosphärischen Plasmastrahls vorgesehen werden, in die der in der Lüftungsanlage geleitete Luftstrom, insbesondere der Luftstrom 14 oder 54, als Arbeitsgas geleitet wird. Auf diese Weise kann die Ozonkonzentration im Arbeitsgas und damit in dem in der Lüftungsanlage geleiteten Luftstrom reduziert werden, vorzugsweise auf eine Konzentration unterhalb von 0.1 ppm.

Figur 4 zeigt ein Ausführungsbeispiel einer solchen Plasmadüse.

Die Plasmadüse 130 weist ein Düsenrohr 132 aus Metall auf, das sich im Wesentlichen konisch zu einer Düsenrohrmündung 134 verjüngt. An dem der Düsenrohrmündung 134 entgegengesetztem Ende weist das Düsenrohr 132 eine Dralleinrichtung 136 mit einem Einlass 138 für ein Arbeitsgas auf.

Eine Zwischenwand 140 der Dralleinrichtung 136 weist einen Kranz von schräg in Umfangsrichtung angestellten Bohrungen 142 auf, durch die das Arbeitsgas verdrallt wird. Der stromabwärtige, konisch verjüngte Teil des Düsenrohrs 132 wird deshalb von dem Arbeitsgas in Form eines Wirbels 144 durchströmt, dessen Kern auf der Längsachse des Düsenrohrs 132 verläuft.

An der Unterseite der Zwischenwand 140 ist mittig eine Elektrode 146 angeordnet, die koaxial in Richtung des verjüngten Abschnitts in das Düsenrohr 132 hineinragt. Die Elektrode 146 ist elektrisch mit der Zwischenwand 140 und den übrigen Teilen der Dralleinrichtung 136 verbunden. Die Dralleinrichtung 136 ist durch ein Keramikrohr 148 elektrisch gegen das Düsenrohr 132 isoliert. Über die Dralleinrichtung 136 wird an die Elektrode 146 eine hochfrequente Hochspannung angelegt, die von einem Transformator 150 erzeugt wird. Der Einlass 138 ist insbesondere derart angeordnet, dass ein Teil des Luftstroms 14 bzw. 54 in die Plasmadüse 130 gelangen kann. Das Düsenrohr 132 ist geerdet. Durch die angeregte Spannung wird eine Hochfrequenzentladung in Form eines Lichtbogens 152 zwischen der Elektrode 146 und dem Düsenrohr 132 erzeugt.

Die Begriffe "Lichtbogen" bzw. "Bogenentladung" werden vorliegend als phänomenologische Beschreibung der Entladung verwendet, da die Entladung in Form eines Lichtbogens auftritt. Der Begriff "Lichtbogen" wird anderweitig auch als Entladungsform bei Gleichspannungsentladungen mit im Wesentlichen konstanten Spannungswerten verwendet. Vorliegend handelt es sich jedoch um eine Hochfrequenzentladung in Form eines Lichtbogens, also um eine hochfrequente Bogenentladung.

Aufgrund der drallförmigen Strömung des Arbeitsgases wird dieser Lichtbogen 152 im Wirbelkern auf der Achse des Düsenrohrs 132 kanalisiert, so dass er sich erst im Bereich der Düsenrohrmündung 134 zur Wand des Düsenrohrs 132 verzweigt.

Das Arbeitsgas, das im Bereich des Wirbelkerns und damit in unmittelbarer Nähe des Lichtbogens 152 mit hoher Strömungsgeschwindigkeit rotiert, kommt mit dem Lichtbogen 152 in innige Berührung und wird dadurch zum Teil in den Plasmazustand überführt, so dass ein atmosphärischer Plasmastrahl 154 durch die Düsenrohrmündung 134 aus der Plasmadüse 130 austritt.

Es hat sich herausgestellt, dass sich die Ozonkonzentration in einem Gas dadurch erheblich verringern lässt, dass das Gas als Arbeitsgas in die Plasmadüse 130 geleitet wird. Durch die Anregung des Arbeitsgases mittels der Entladung 152 werden Ozonmoleküle aufgespalten bzw. in andere Moleküle umgewandelt, so dass die Ozonkonzentration des aus der Plasmadüse 130 austretenden Arbeitsgases bzw. Plasmastrahls geringer ist als die Ozonkonzentration des in die Plasmadüse 130 hinein geführten Arbeitsgases.

Damit lässt sich die in Figur 4 dargestellte Plasmadüse 130 in der Lüftungsanlage 10 bzw. 50 vorteilhaft einsetzen, um am Ende eines Reinigungsbetriebs die Ozonkonzentration zu reduzieren und dadurch eine Gesundheits- bzw. Geruchsbeeinträchtigung durch das Ozon zu vermeiden.

Zusätzlich oder alternativ zu einer solchen Plasmadüse können in der Lüftungsanlage 10 auch Aktivkohlefilter und/oder Keramikfilter (nicht dargestellt) installiert sein, um die Ozonkonzentration am Ende eines Reinigungsbetriebs zu reduzieren.

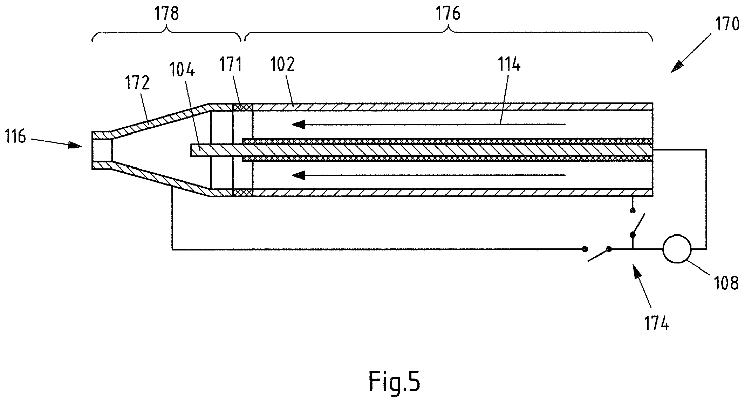

Eine Plasmadüse zur Erzeugung eines atmosphärischen Plasmastrahls lässt sich in vorteilhafter Weise auch in die Erzeugungseinheit integrieren. Figur 5 zeigt ein Ausführungsbeispiel einer solchen Erzeugungseinheit 170, deren Aufbau der Erzeugungseinheit 100 aus Figur 3 ähnelt. Gleiche Komponenten sind jeweils mit gleichen Bezugszeichen versehen.

Die Erzeugungseinheit 170 unterscheidet sich von der Erzeugungseinheit 100 dadurch, dass die Innenelektrode 104 an dem den Gasauslass 116 zugewandten Ende über die dielektrische Schicht 106 hinaus verlängert ist und eine zweite, von der ersten Außenelektrode 102 durch einen Isolator 171 elektrisch isolierte und sich im Bereich des Gasauslasses 116 zu einer Düsenöffnung verjüngende Außenelektrode 172 aufweist. Die zweite Außenelektrode 172 ist ebenfalls an die Spannungsquelle 108 angeschlossen. Über vorgesehene Schalter 174 können jeweils die erste und/oder die zweite Außenelektrode 102, 172 beschaltet werden.

Die Erzeugungseinheit 170 weist damit einen ersten Erzeugungsabschnitt 176 auf, der zur Erzeugung reaktiver Sauerstoffspezies eingerichtet ist, sowie einen zweiten Erzeugungsabschnitt 178, der - vergleichbar mit der Plasmadüse 130 in Fig. 4 - zur Erzeugung eines atmosphärischen Plasmastrahls eingerichtet ist. Mit dem zweiten Erzeugungsabschnitt 178 kann die Ozonkonzentration in dem Arbeitgas 114 reduziert werden.

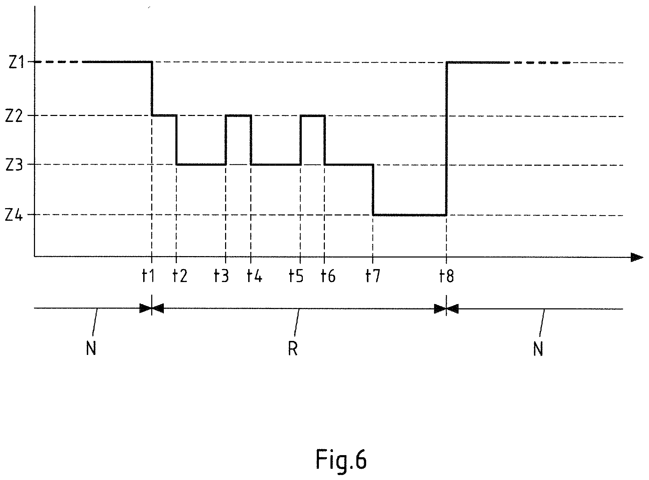

Figur 6 zeigt ein Steuerungsdiagramm entsprechend eines Ausführungsbeispiels des Verfahrens zum Betrieb einer Lüftungsanlage, insbesondere der Lüftungsanlage 10 oder 50.

In dem Diagramm sind vier Zustände Z1 bis Z4 der jeweils zu steuernden Lüftungsanlage aufgetragen, zwischen denen mittels der Steuerung der Lüftungsanlage, insbesondere der Steuerung 36 bzw. 72, zu vorgegebenen Zeiten t1 bis t8 umgeschaltet wird.

Der Zustand Z1 entspricht einem Normalbetrieb und die Zustände Z2 bis Z4 entsprechen verschiedenen Zuständen im Reinigungsbetrieb der Lüftungsanlage. Die Bedeutung der einzelnen Zustände Z1 bis Z4 wird im Folgenden beschrieben.
Z1: In diesem Zustand läuft die Lüftungsanlage im Normalbetrieb, so dass ein im Leitungssystem geleiteter Luftstrom über einen Auslass des Leitungssystems zur Versorgung eines Raums bereitgestellt wird.
Z2: In diesem Zustand steuert die Steuerung die Erzeugungseinheit der Lüftungsanlage an, so dass diese reaktive Sauerstoffspezies erzeugt und in einen im Leitungssystem der Lüftungsanlage geleiteten Luftstrom einbringt. Dieser Zustand wird auch als Aktivierungszeitraum bezeichnet.
Z3: In diesem Zustand ist die Erzeugungseinheit ausgeschaltet, so dass keine weiteren reaktiven Sauerstoffspezies erzeugt werden. Dieser Zustand wird auch als Abklingzeitraum bezeichnet.
Z4: In diesem Zustand ist eine in die Lüftungsanlage integrierte Plasmadüse aktiviert, so dass die Ozonkonzentration des in dem Leitungssystem geleiteten Luftstroms reduziert wird. Die Erzeugungseinheit ist in diesem Zustand vorzugsweise deaktiviert.

Das Diagramm in Fig. 6 zeigt nun einen exemplarischen Ablauf einer Steuerung der Lüftungsanlage zwischen den oben genannten Zuständen Z1 - Z4.

Die Steuerung kann demnach so eingerichtet sein, dass sie die Lüftungsanlage zu einem vorgegebenen Zeitpunkt t1 von einem Normalbetrieb N in einen Reinigungsbetrieb R umschaltet. Der Reinigungsbetrieb dauert vorliegend vom Zeitpunkt t1 bis zum Zeitpunkt t8 an und kann beispielsweise eine Dauer von ein bis zwei Stunden umfassen. Das Umschalten vom Normalbetrieb N in den Reinigungsbetrieb erfolgt vorzugsweise zu einem Zeitpunkt, zu dem in dem durch die Lüftungsanlage versorgten Raum nicht gearbeitet wird, beispielsweise nachts.

Zum Zeitpunkt t1 schaltet die Steuerung das Leitungssystem vorzugsweise in den Umluftmodus, so dass ein Luftstrom innerhalb des Leitungssystems zirkulieren kann. Dann betreibt die Steuerung die Erzeugungseinheit vom Zeitpunkt t1 bis t7 vorzugsweise phasenweise, wobei die Erzeugungseinheit jeweils für Aktivierungszeiträume (von t1 bis t2, von t3 bis t4 und von t5 bis t6) aktiviert wird, so dass diese reaktive Sauerstoffspezies erzeugt und in den Luftstrom im Leitungssystem einbringt, und wobei die Erzeugungseinheit in Abklingzeiträumen dazwischen (von t2 bis t3, von t4 bis t5 und von t6 bis t7) ausgeschaltet ist.

Zum Ende des Reinigungsbetriebs schaltet die Steuerung die Lüftungsanlage in einen Neutralisierungsbetrieb (von t7 bis t8), bei dem die Erzeugungseinheit abgeschaltet und die Plasmadüse zur Erzeugung eines atmosphärischen Plasmastrahls angeschaltet wird, um die Ozonkonzentration innerhalb der Lüftungsanlage zu reduzieren.

Am Ende des Reinigungsbetriebs zum Zeitpunkt t8 schaltet die Steuerung die Lüftungsanlage automatisch wieder auf Normalbetrieb um, so dass die Lüftungsanlage weiter läuft.

Durch diese Betriebsweise wird ermöglicht, dass die Lüftungsanlage regelmäßig und automatisch desinfiziert bzw. sterilisiert wird, ohne dass hierzu langwierige Wartungsarbeiten erforderlich sind.

## Patentansprüche

1. Lüftungsanlage (10, 50),
- mit einem Leitungssystem (12, 52), das dazu eingerichtet ist, einen Luftstrom (14, 54) zu leiten und den Luftstrom (14, 54) während eines Normalbetriebs (N) über einen Auslass (16, 56) des Leitungssystems (12, 52) zur Versorgung eines Raums (18, 58) bereitzustellen,
- wobei die Lüftungsanlage (10, 50) als Klimaanlage ausgebildet ist und einen Wärmetauscher (64) aufweist, der dazu eingerichtet ist, während des Normalbetriebs (N) einen in dem Leitungssystem (12, 52) geleiteten Luftstrom (14, 54) zu kühlen, wobei die Lüftungsanlage (10, 50) eine Erzeugungseinheit (34, 66,100,170) aufweist, die dazu eingerichtet ist, während eines Reinigungsbetriebs (R) reaktive Sauerstoffspezies zu erzeugen und diese dem im Leitungssystem (12, 52) geleiteten Luftstrom (14, 54) zuzuführen,
- **dadurch gekennzeichnet, dass** die Erzeugungseinheit (34, 66, 100, 170) dazu eingerichtet ist, reaktive Sauerstoffspezies mittels einer elektrischen Entladung (110) in einem Arbeitsgas (114) zu erzeugen, vorzugsweise mittels einer dielektrisch behinderten Entladung, und
- dass die Erzeugungseinheit (34, 66, 100, 170) derart angeordnet und eingerichtet ist, dass mit der Erzeugungseinheit (34, 66, 100, 170) erzeugte reaktive Sauerstoffspezies zum Wärmetauscher (64) gelangen.

2. Lüftungsanlage nach Anspruch 1,
**dadurch gekennzeichnet,**
**dass** die Lüftungsanlage (10, 50) eine Steuerung (36, 68) aufweist, die dazu eingerichtet ist, die Erzeugungseinheit (34, 66, 100, 170) derart zu steuern, dass während eines Reinigungsbetriebs (R) in einem im Leitungssystem (12, 52) geleiteten Luftstrom (14, 54) eine Konzentration an reaktiven Sauerstoffspezies, insbesondere an Ozon, von mindestens 7 g/m³, vorzugsweise mindestens 10 g/m³ erreicht wird.

3. Lüftungsanlage nach Anspruch 1 oder 2,
**dadurch gekennzeichnet,**
**dass** die Lüftungsanlage (10, 50) eine Steuerung (36, 68) aufweist, die dazu eingerichtet ist, die Lüftungsanlage (10, 50) zu vorgegebenen Zeiten und/oder für vorgegebene Dauern im Reinigungsbetrieb (R) zu betreiben.

4. Lüftungsanlage nach Anspruch 3,
**dadurch gekennzeichnet,**
**dass** die Steuerung (36, 68) dazu eingerichtet ist, die Erzeugungseinheit (34, 66, 100,170) während des Reinigungsbetriebs (R) phasenweise zu betreiben.

5. Lüftungsanlage nach einem der Ansprüche 1 bis 4,
**dadurch gekennzeichnet,**
**dass** das Leitungssystem (12, 52) zwischen einem Zuluftmodus und einem Umluftmodus umschaltbar ist, wobei das Leitungssystem (12, 52) im Zuluftmodus dazu eingerichtet ist, einen im Leitungssystem (12, 52) geleiteten Luftstrom (14, 54) über den Auslass (16, 56) zur Versorgung eines Raums (18, 58) bereitzustellen, und wobei das Leitungssystem (12, 52) im Umluftmodus dazu eingerichtet ist, einen im Leitungssystem (12, 52) geleiteten Luftstrom (14, 54) innerhalb des Leitungssystems (12, 52) zu zirkulieren.

6. Lüftungsanlage nach einem der Ansprüche 1 bis 5,
**dadurch gekennzeichnet,**
- **dass** die Lüftungsanlage (10, 50) eine Vorrichtung zur Reduzierung einer Ozon-Konzentration umfasst, die dazu eingerichtet ist, die Ozon-Konzentration des im Leitungssystem (12, 52) geleiteten Luftstroms (14, 54) zu reduzieren,
- **dass** die Lüftungsanlage (10, 50) als Vorrichtung zur Reduzierung einer Ozon-Konzentration eine Plasmadüse (130) zur Erzeugung eines atmosphärischen Plasmastrahls (154) umfasst, wobei die Plasmadüse (130) dazu eingerichtet ist, den Plasmastrahl (154) mittels Erzeugung einer Bogenentladung durch Anlegen einer hochfrequenten Hochspannung zwischen zwei Elektroden in einem Arbeitsgas zu erzeugen,
- **dass** das Leitungssystem (12, 52) und die Plasmadüse (130) derart eingerichtet sind, dass der in dem Leitungssystem (12, 52) geleitete Luftstrom (14, 54) der Plasmadüse (130) als Arbeitsgas zuführbar ist.

7. Lüftungsanlage nach einem der Ansprüche 1 bis 6,
**dadurch gekennzeichnet,**
**dass** die Lüftungsanlage (10, 50) eine Steuerung (36, 68) aufweist, die dazu eingerichtet ist, die Plasmadüse (130) für einen vorgegebenen Zeitraum am Ende eines Reinigungsbetriebs (R) zu betreiben.

8. Lüftungsanlage nach einem der Ansprüche 1 bis 7,
**dadurch gekennzeichnet,**
- **dass** die Lüftungsanlage (10, 50) eine Mehrzahl von Erzeugungseinheiten (34, 66, 100, 170) aufweist, die jeweils dazu eingerichtet sind, während eines Reinigungsbetriebs (R) reaktive Sauerstoffspezies zu erzeugen und die erzeugten reaktiven Sauerstoffspezies einem im Leitungssystem (12, 52) geleiteten Luftstrom zuzuführen, und
- **dass** die Lüftungsanlage (10, 50) eine Steuerung (36, 68) aufweist, die dazu eingerichtet ist, die Erzeugungseinheiten (34, 66, 100, 170) während eines Reinigungsbetriebs (R) abwechselnd zu betreiben.

9. Lüftungsanlage nach einem der Ansprüche 1 bis 8,
**dadurch gekennzeichnet,**
**dass** die Lüftungsanlage (10, 50) eine Steuerung (36, 68) aufweist, die dazu eingerichtet ist, ein Verfahren nach einem der Ansprüche 12 bis 14 durchzuführen.

10. Lüftungsanlage nach einem der Ansprüche 1 bis 9,
**dadurch gekennzeichnet,**
**dass** der Auslass des Leitungssystems (12, 52) an eine Lüftungsöffnung eines Raumes (18, 58) angeschlossen ist, vorzugsweise eines Raumes eines Gebäudes, insbesondere eines Operationssaals, eines Quarantäne-Raums auf einer Isolierstation, eines Reinraums oder eines Laborraums, oder eines Innenraumes eines geschlossenen Geräts, insbesondere eines Laborgeräts oder eines Haushaltsgeräts.

11. Verwendung einer Lüftungsanlage (10, 50) nach einem der Ansprüche 1 bis 10 zur Belüftung und/oder Klimatisierung eines Raumes eines Gebäudes, insbesondere eines Operationssaals, eines Quarantäne-Raums auf einer Isolierstation, eines Reinraums oder eines Laborraums, oder eines Innenraumes eines geschlossenen Geräts, insbesondere eines Laborgeräts oder eines Haushaltsgeräts.

12. Verfahren zum Betrieb einer Lüftungsanlage (10, 50) nach einem der Ansprüche 1 bis 10, insbesondere einer Klimaanalage,
- bei dem ein Luftstrom (14, 54) in dem Leitungssystem (12, 52) geleitet und während eines Normalbetriebs (N) über den Auslass (16, 56) zur Versorgung eines Raums (18, 58) bereitgestellt wird, insbesondere einem Raum (18, 58) zugeführt wird, und
- bei dem während eines Reinigungsbetriebs (R) reaktive Sauerstoffspezies mit der Erzeugungseinheit (34, 66, 100, 170) erzeugt und einem im Leitungssystem (12, 52) geleiteten Luftstrom (14, 54) zugeführt werden.

13. Verfahren nach Anspruch 12,
**dadurch gekennzeichnet,**
**dass** während des Reinigungsbetriebs (R) der Luftstrom (14, 54) innerhalb des Leitungssystems (12, 52) zirkuliert wird.

14. Verfahren nach Anspruch 12 oder 13,
**dadurch gekennzeichnet,**
- **dass** während eines ersten Zeitabschnitts des Reinigungsbetriebs (R) reaktive Sauerstoffspezies mit der Erzeugungseinheit (34, 66, 100, 170) erzeugt und einem im Leitungssystem (12, 52) geleiteten Luftstrom (14, 54) zugeführt werden und
- **dass** während eines zweiten Zeitabschnitts des Reinigungsbetriebs (R) der im Leitungssystem (12, 52) geleitete Luftstrom (14, 54) zumindest teilweise einer Plasmadüse (130) zur Erzeugung eines atmosphärischen Plasmastrahls (154) als Arbeitsgas zugeführt wird.

15. Verwendung einer Erzeugungseinheit (34, 66, 170) zur Erzeugung reaktiver Sauerstoffspezies für eine Lüftungsanlage (10, 50) nach einem der Ansprüche 1 bis 10,
- mit einem Gaseinlass (112), der zur Einleitung eines Arbeitsgases (114) eingerichtet ist, und
- mit einem Gasauslass (116), der zum Auslass des Arbeitsgases (114) bzw. der reaktiven Sauerstoffspezies eingerichtet ist,
- wobei die Erzeugungseinheit (34, 66, 170) dazu eingerichtet ist, während eines Erzeugungsbetriebs reaktive Sauerstoffspezies mittels einer elektrischen Entladung (110) in einem durch den Gaseinlass (112) eingeleiteten Arbeitsgas (114) zu erzeugen, vorzugsweise mittels einer dielektrisch behinderten Entladung,
**dadurch gekennzeichnet**
- **dass** die Erzeugungseinheit (34, 66, 170) weiterhin dazu eingerichtet ist, während eines Neutralisierungsbetriebs einen atmosphärischen Plasmastrahl (154) mittels Erzeugung einer Bogenentladung durch Anlegen einer hochfrequenten Hochspannung zwischen zwei Elektroden (104, 172) in dem durch den Gaseinlass (112) eingeleiteten Arbeitsgas (114) zu erzeugen.

## Claims

1. Ventilation system (10, 15),
- having a piping system (12, 52) which is configured to convey an air flow (14, 54) and to provide the air flow (14, 54) via an outlet (16, 56) of the piping system (12, 52) for supplying a room (18, 58) during a normal operation (N),
- wherein the ventilation system (10, 15) is designed as an air conditioning system and comprises a heat exchanger (64), which is configured to cool an air flow (14, 54) conveyed in the piping system (12, 52) during the normal operation (N),
- wherein the ventilation system (10, 50) comprises a generating unit (34, 66, 100, 170), which is configured to generate reactive oxygen species during a cleaning operation (R) and to feed these to the air flow (14, 54) conveyed in the piping system (12, 52),
**characterised in**
- **that** the generation unit (34, 66, 100, 170) is configured to generate reactive oxygen species by means of an electrical discharge (110) in a working gas (114), preferably by means of a dielectric barrier discharge, and
- **that** the generating unit (34, 66, 100, 170) is arranged and configured in such a way that reactive oxygen species generated with the generating unit (34, 66, 100, 170) reach the heat exchanger (64).

2. Ventilation system according to Claim 1,
**characterised in that** the ventilation system (10, 50) comprises a control (36, 68), which is configured to control the generating unit (34, 66, 100, 170) in such a way that during a cleaning operation (R) a concentration of reactive oxygen species, in particular of ozone, of at least 7 g/m³ and preferably of at least 10 g/m³, is achieved in an air flow (14, 54) conveyed in the piping system (12, 52).

3. Ventilation system according to Claim 1 or 2,
**characterised in that** the ventilation system (10, 50) comprises a control (36, 68), which is configured to operate the ventilation system (10, 50) in the cleaning operation (R) at predetermined times and/or for predetermined periods.

4. Ventilation system according to Claim 3,
**characterised in that** the control (36, 68) is configured to operate the generating unit (34, 66, 100, 170) in phases during the cleaning operating (R).

5. Ventilation system according to any one of Claims 1 to 4,
**characterised in that**
the piping system (12, 52) is switchable between an air supply mode and an air circulating mode, wherein the piping system (12, 52) in the air supply mode is configured to provide an air flow (14, 54) conveyed in the piping system (12, 52) via the outlet (16, 56) for supplying a room (18, 58), and wherein the piping system (12, 52) in the air circulating mode is configured to circulate an air flow (14, 54) conveyed in the piping system (12, 52) within the piping system (12, 52).

6. Ventilation system according to any one of Claims 1 to 5,
**characterised in**
- **that** the ventilation system (10, 50) comprises a device for reducing an ozone concentration, which is configured to reduce the ozone concentration of the air flow (14, 54) conveyed in the piping system (12, 52),
- **that** the ventilation system (10, 50) comprises, as the device for reducing an ozone concentration, a plasma nozzle (130) for generating an atmospheric plasma jet (154), wherein the plasma nozzle (130) is configured to generate the plasma jet (154) by means of generating an arc discharge by applying a high-frequency high voltage between two electrodes in a working gas,
- **that** the piping system (12, 52) and the plasma jet (130) are configured in a way that the air stream (14, 54) conveyed in the piping system (12, 52) can be fed as working gas to the plasma nozzle (130).

7. Ventilation system according to any one of Claims 1 to 6,
**characterised in that** the ventilation system (10, 50) comprises a control (36, 68), which is configured to operate the plasma nozzle (130) for a predetermined period of time at the end of a cleaning operation (R).

8. Ventilation system according to any one of Claims 1 to 7,
**characterised in**
- **that** the ventilation system (10, 50) comprises a plurality of generating units (34, 66, 100, 170) which are each configured to generate reactive oxygen species and to feed the generated reactive oxygen species to an air flow conveyed in the piping system (12, 52) during a cleaning operation (R), and
- **that** the ventilation system (10, 50) comprises a control (36, 68), which is configured to operate the generating units (34, 66, 100, 170) in an alternating manner during a cleaning operation (R).

9. Ventilation system according to any one of Claims 1 to 8,
**characterised in that** the ventilation system (10, 50) comprises a control (36, 68), which is configured to carry out a method according to any one of Claims 12 to 14.

10. Ventilation system according to any one of Claims 1 to 9,
**characterised in that** the outlet of the piping system (12, 52) is attached to a ventilation opening of a room (18, 58), preferably a room of a building, in particular an operating theatre, a quarantine room on an isolation ward, a clean room or a laboratory room, or an interior of a closed device, in particular a laboratory device or a household appliance.

11. Use of a ventilation system (10, 50) according to any one of Claims 1 to 10 for the ventilation and/or air conditioning of a room of a building, in particular an operating theatre, a quarantine room on an isolation ward, a clean room or a laboratory room, or an interior of a closed device, in particular a laboratory device or a household appliance.

12. Method for operating a ventilation system (10, 50) according to any one of Claims 1 to 10, in particular an air conditioning system,
- in which an air flow (14, 24) is conveyed in the piping system (12, 52) and is provided via the outlet (16, 56) for supplying a room (18, 58), in particular is fed to a room (18, 58), during a normal operation (N), and
- in which during a cleaning operation (R) reactive oxygen species are generated with the generating unit (34, 66, 100, 170) and are fed to an air flow (14, 54) conveyed in the piping system (12, 52).

13. Method according to Claim 12,
**characterised in that** during the cleaning operation (R) the air flow (14, 54) is circulated within the piping system (12, 52).

14. Method according to Claim 12 or 13, **characterised in**
- **that** during a first time period of the cleaning operation (R) reactive oxygen species are generated with the generating unit (34, 66, 100, 170) and are fed to an air flow (14, 54) conveyed in the piping system, and
- **that** during a second time period of the cleaning operation (R) the air flow (14, 54) conveyed in the piping system (12, 52) is at least partially fed to a plasma nozzle (130) for generating an atmospheric plasma jet (154) as working gas.

15. Use of a generating unit (34, 66, 100, 170) for generating reactive oxygen species for a ventilation system (10, 50) according to any one of Claims 1 to 10,
- with a gas inlet (112) which is configured to feed in a working gas (114), and
- with a gas outlet (116) which is configured to release the working gas (114) and/or the reactive oxygen species,
- wherein the generating unit (34, 66, 100, 170) is configured to generate a reactive oxygen species during a generation operation by means of an electrical discharge in a working gas (114) fed in through the gas inlet (112), preferably by means of a dielectric barrier discharge,
**characterised in**
- **that** the generating unit (34, 66, 100, 170) is furthermore configured to generate an atmospheric plasma jet (154) during a neutralisation operation by generating an arc discharge by applying a high-frequency high voltage between two electrodes (104, 172) in the working gas (114) fed in through the gas inlet (112).

## Revendications

1. Dispositif de ventilation (10, 50),
- avec un système de conduits (12, 52) conçu pour guider un flux d'air (14, 54) et pour mettre à disposition le flux d'air (14, 54) pour l'approvisionnement d'une pièce (18, 58), lors d'un fonctionnement normal (N), par le biais d'une sortie (16, 56) du système de conduits (12, 52),
- où le dispositif de ventilation (10, 50) est conçu en tant que climatisation et présente un échangeur thermique (64), lequel échangeur thermique est conçu, lors d'un fonctionnement normal (N), de sorte à refroidir un flux d'air (14, 54) guidé par le système de conduits (12, 52),
- où le dispositif de ventilation (10, 50) comporte une unité de production (34, 66, 100, 170) conçue de sorte à produire, lors d'un fonctionnement en mode nettoyage (R), des espèces d'oxygène réactives, et de sorte à introduire celles-ci dans le flux d'air (14, 54) guidé par le système de conduits (12, 52),
**caractérisé en ce que**
- l'unité de production (34, 66, 100, 170) est conçue de sorte à produire des espèces d'oxygène réactives à l'aide d'une décharge électrique (110) dans un gaz de travail (114), de préférence au moyen d'une décharge à barrière diélectrique, et
- l'unité de production (34, 66, 100, 170) est agencée et conçue de telle sorte que des espèces d'oxygène réactives produites avec l'unité de production (34, 66, 100, 170) parviennent à l'échangeur thermique (64).

2. Dispositif de ventilation selon la revendication 1,
**caractérisé en ce que**
le dispositif de ventilation (10, 50) présente une commande (36, 68) conçue pour commander l'unité de production (34, 66, 100, 170) de telle sorte que, lors d'un fonctionnement en mode nettoyage (R), une concentration d'espèces d'oxygène réactives dans un flux d'air (14, 54) guidé par le système de conduits (12, 52), notamment d'ozone, d'au moins 7 g/m³, de préférence au moins 10 g/m³ soit atteinte.

3. Dispositif de ventilation selon la revendication 1 ou 2,
**caractérisé en ce que**
le dispositif de ventilation (10, 50) présente une commande (36, 68) conçue de sorte à faire fonctionner le dispositif de ventilation (10, 50) à des heures prédéfinies et/ou pendant des durées prédéterminées en fonctionnement en mode nettoyage (R).

4. Dispositif de ventilation selon la revendication 3,
**caractérisé en ce que**
la commande (36, 68) est conçue de sorte à faire fonctionner l'unité de production (34, 66, 100, 170) par phases lors d'un fonctionnement en mode nettoyage (R).

5. Dispositif de ventilation selon l'une des revendications 1 à 4,
**caractérisé en ce que**
le système de conduits (12, 52) peut être commuté entre un mode air entrant et un mode recyclage d'air, où le système de conduits (12, 52) est conçu de sorte à mettre à disposition, dans le mode air entrant, pour l'approvisionnement d'une pièce (18, 58), par le biais d'une sortie (16, 56), un flux d'air (14, 54) guidé par le système de conduits (12, 52), et où le système de conduits (12, 52) est conçu, dans le mode recyclage d'air, de sorte à faire circuler un flux d'air (14, 54) guidé par le système de conduits (12, 52) à l'intérieur du système de conduits (12, 52).

6. Dispositif de ventilation selon l'une des revendications 1 à 5,
**caractérisé en ce que**
- le système de ventilation (10, 15) comporte un dispositif de réduction d'une concentration d'ozone, lequel dispositif de réduction étant conçu de sorte à réduire la concentration en ozone du flux d'air (10, 54) guidé par le système de conduits (12, 52),
- le dispositif de ventilation (10, 50) comporte, en tant que dispositif de réduction d'une concentration d'ozone, une buse à plasma (130) pour la production d'un faisceau de plasma atmosphérique (154), où la buse à plasma (130) est conçue de sorte à produire le faisceau de plasma (154) à l'aide de la production d'une décharge électrique en arc par l'application d'une haute tension à haute fréquence entre deux électrodes dans un gaz de travail,
- le système de conduits (12, 52) et la buse à plasma (130) sont conçus de telle sorte que le flux d'air (14, 54) guidé par le système de conduits (12, 52) puisse être amené à la buse à plasma (130).

7. Dispositif de ventilation selon l'une des revendications 1 à 6,
**caractérisé en ce que**
le dispositif de ventilation (10, 50) présente une commande (36, 68) conçue de sorte à faire fonctionner la buse à plasma (130) pendant un laps de temps prédéterminé à la fin du fonctionnement en mode nettoyage (R).

8. Dispositif de ventilation selon l'une des revendications 1 à 7,
**caractérisé en ce que**
le dispositif de ventilation (10, 50) présente une multitude d'unités de production (34, 66, 100,170) qui sont chacune conçues de sorte à produire, lors d'un fonctionnement en mode nettoyage (R), des espèces d'oxygène réactives et à introduire les espèces d'oxygène réactives produites dans un flux d'air guidé par le système de conduits (12, 52), et
le dispositif de ventilation (10, 50) présente une commande (36, 68) conçue de sorte à faire fonctionner l'unité de production (34, 66, 100, 170) de manière alternative lors d'un fonctionnement en mode nettoyage (R).

9. Dispositif de ventilation selon l'une des revendications 1 à 8,
**caractérisé en ce que**
le dispositif de ventilation (10, 50) présente une commande (36, 68) conçue de sorte à mettre en oeuvre un procédé selon l'une des revendications 12 à 14.

10. Dispositif de ventilation selon l'une des revendications 1 à 9,
**caractérisé en ce que**
la sortie du système de conduits (12, 52) est raccordée à une ouverture de ventilation d'une pièce (18, 58), de préférence d'une pièce d'un bâtiment, notamment d'une salle d'opération, d'un espace de quarantaine dans une station d'isolation, d'une salle blanche ou d'un laboratoire, ou d'un compartiment interne d'un appareil fermé, notamment d'un appareil de laboratoire ou d'un appareil électroménager.

11. Utilisation d'un dispositif de ventilation (10, 50) selon l'une des revendications 1 à 10 pour l'aération et/ou la climatisation d'une pièce d'un bâtiment, notamment d'une salle d'opération, d'un espace de quarantaine dans une station d'isolation, d'une salle blanche ou d'un laboratoire, ou d'un compartiment interne d'un appareil fermé, notamment d'un appareil de laboratoire ou d'un appareil électroménager.

12. Procédé de fonctionnement d'un dispositif de ventilation (10, 50) selon l'une des revendications 1 à 10, notamment d'une climatisation,
- dans lequel un flux d'air (14, 54) est guidé par le système de conduits (12, 52) et est mis à disposition, lors d'un fonctionnement normal (N), pour l'approvisionnement d'une pièce (18, 58), par le biais de la sortie (16, 56), notamment est introduit dans une pièce (18, 58), et
- dans lequel, lors d'un fonctionnement en mode nettoyage (R), des espèces d'oxygène réactives sont produites avec l'unité de production (34, 66, 100, 170) et sont introduites dans un flux d'air (14, 54) guidé par le système de conduits (12, 52).

13. Procédé selon la revendication 12,
**caractérisé en ce que**,
lors d'un fonctionnement en mode nettoyage (R), le flux d'air (14, 54) circule à l'intérieur du système de conduits (12, 52).

14. Procédé selon la revendication 12 ou 13,
**caractérisé en ce que**
- pendant une première période du fonctionnement en mode nettoyage (R) des espèces d'oxygène réactives sont produites avec l'unité de production (34, 66, 100, 170) et sont introduites dans un flux d'air (14, 54) guidé par le système de conduits (12, 52) et
- pendant une deuxième période du fonctionnement en mode nettoyage (R), le flux d'air (14, 54) guidé par le système de conduits (12, 52) est acheminé au moins partiellement à une buse de plasma (130) pour la production d'un faisceau de plasma atmosphérique (154), en tant que gaz de travail.

15. Utilisation d'une unité de production (34, 66, 170) pour produire des espèces d'oxygène réactives pour un dispositif de ventilation (10, 50) selon l'une des revendications 1 à 10,
- avec une entrée de gaz (112) conçue pour l'introduction d'un gaz de travail (114), et
- avec une sortie de gaz (116) conçue pour la sortie du gaz de travail (114) ou des espèces d'oxygène réactives,
- où l'unité de production (34, 66, 170) est conçue de sorte à produire, lors d'un fonctionnement en mode production, des espèces d'oxygène réactives à l'aide d'une décharge électrique (110) dans un gaz de travail (114) introduit à travers l'entrée de gaz (112), de préférence à l'aide d'une décharge à barrière diélectrique,
**caractérisée en ce que**
- l'unité de production (34, 66, 170) est en outre conçue de sorte à produire, lors d'un fonctionnent en mode de neutralisation, un faisceau de plasma atmosphérique (154) à l'aide de la production d'une décharge électrique en arc par l'application d'une haute tension à haute fréquence entre deux électrodes (104, 172) dans le gaz de travail (114) introduit à travers l'entrée de gaz (112).
